(19) Europäisches Patentamt
European Patent Office
Office européen des brevets

(11) Publication number: **0 380 144 B1**

# (12) EUROPEAN PATENT SPECIFICATION

(45) Date of publication of patent specification: **19.01.94**

(51) Int. Cl.[5]: **C07C 311/37**, C07C 303/40

(21) Application number: **90104584.9**

(22) Date of filing: **21.07.87**

(60) Publication number of the earlier application in accordance with Art.76 EPC: **0 257 787**

## (54) Process for producing optically active benzene-sulfonamide derivatives.

(30) Priority: **21.07.86 JP 172285/86**

(43) Date of publication of application: **01.08.90 Bulletin 90/31**

(45) Publication of the grant of the patent: **19.01.94 Bulletin 94/03**

(84) Designated Contracting States: **AT DE ES FR GB IT**

(56) References cited:
**EP-A- 0 034 432**
**EP-A- 0 092 787**
**US-A- 4 000 197**

(73) Proprietor: **YAMANOUCHI PHARMACEUTICAL CO. LTD.**
**No. 3-11 Nihonbashi-Honcho, 2-chome**
**Chuo-ku**
**Tokyo(JP)**

(72) Inventor: **Okada, Minoru, Postal Code 305, c/o Central**
**Research Lab.,**
**Yamanouchi Pharmaceuticals Co. Ltd.**
**No.21 Miyukigaoka, Tsukuba-shi, Ibaragi(JP)**
Inventor: **Yoshida, Koichi**
**9-16, Shirokane 3-chome**
**Minato-ku Tokyo(JP)**
Inventor: **Takanobu, Kiyoshi**
**1142-1, Hongo-cho**
**Omiya-shi Saitama(JP)**

(74) Representative: **Geering, Keith Edwin et al**
**REDDIE & GROSE**
**16 Theobalds Road**
**London WC1X 8PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a new process for producing optically active benzenesulfonamide derivatives which are represented by the following general formula (I) and are useful as antihypotensive agents and as intermediates for the manufacture of optically active drugs,

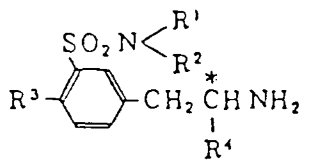

(I)

wherein $R^1$ and $R^2$ are the same or different and selected independently from a hydrogen atom and lower alkyl groups; $R^3$ is a hydrogen atom or a lower alkyl, hydroxyl or lower alkoxyl group; and $R^4$ stands for a lower alkyl group. A similar process is disclosed in US-A-4 000 197 but the presence of a sulfonamide group in the scheme is not contemplated.

Most of the compounds (I) are known from Japanese Patent Publication No.18353 (1983) which discloses compounds of formula (A-I)

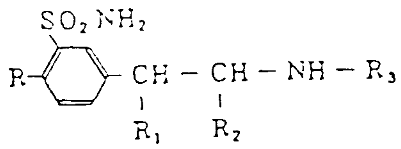

(A-I)

(wherein R is a lower alkyl, lower alkoxyl or hydroxyl group; $R_1$ is a hydrogen or halogen atom or hydroxyl; $R^2$ is a hydrogen atom or a lower alkyl group; and $R_3$ is a hydrogen atom or a lower alkyl group), indicates that all the related optical isomers are included, and states that the compounds (A-I) are useful as antihypotensive agents with no side effects, such as increased heart rate and palpitation.

We disclosed in Japanese Patent publication No.110665 (1981), particularly in Examples 23 and 24, that sulfamoyl-substituted phenetylamine derivatives having adrenergic receptor $\alpha$-blocking effect and useful as hypotensive agents can be prepared from the optically active compounds

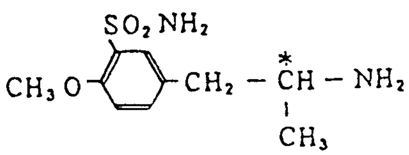

[A-I-a-free, R(-) or S(+)]

Manufacture of these optically active starting materials was not described, but they were in fact prepared by optical resolution of a racemic mixture [a mixture of A-I-a, R(-) and S(+)] obtained by the method described in Japanese Patent publication No.18353 (1983) mentioned above via reaction steps given below:

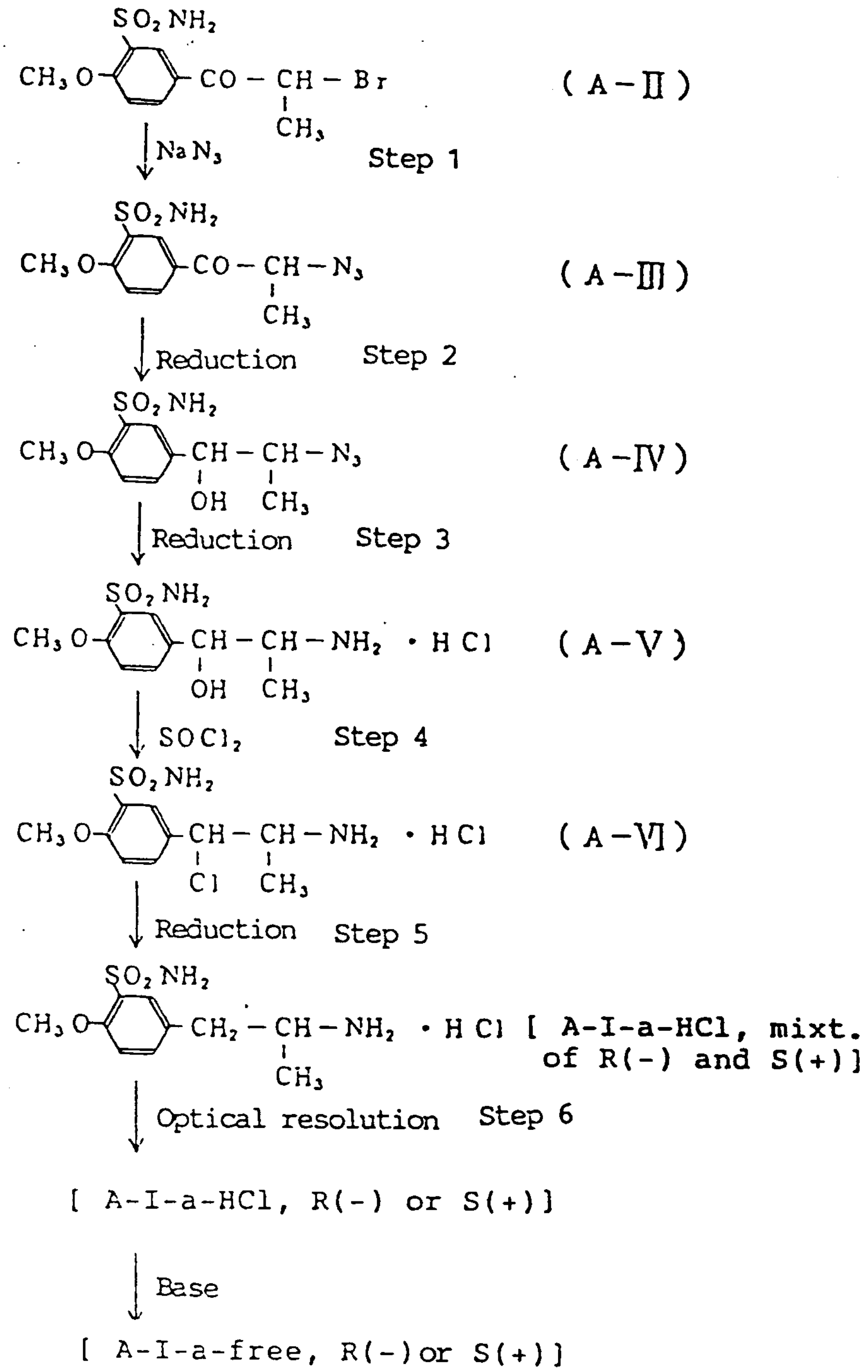


In the prior manufacturing method, salts of compounds (I) are prepared from halo-alkyl sulfamoylphenyl ketone (II) via six reaction steps; this method is cumbersome because of the many reaction steps involved and the low product yield.

When only one optical isomer of a compound (I) is put to use as a drug, the useful yield will be less than half, leading to a further increase in the manufacturing cost.

This disadvantage is more marked when a compound (I) is used as an intermediate for the manufacture of optically active drugs; its practical use is not advantageous, as with another type of intermediate described in Japanese Patent publication No.10665, m-(1-halo-2-substituted-aminoalkyl)-O-substituted-benzenesulfonamides, whose optical resolution is difficult.

Intensive studies in search for a more advantageous method for synthesizing the compounds (I) have led us to find that these can be simply prepared with unusually high reaction and optical yields by

diastereoisomeric separation.

Thus this invention provides a process for producing compounds of formula (I), which comprises decomposing a m-(2-substituted-alkylaminoalkyl)benzenesulfonamide derivative of formula (II)

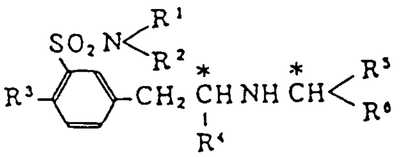

(II)

( wherein $R^1$, $R^2$, $R^3$ and $R^4$ are as defined above; $R^5$ denotes a lower alkyl, a carboxy-lower-alkyl or a lower-alkoxycarbonyl-lower-alkyl group; and $R^6$ is a substituted or unsubstituted phenyl group).

The compounds (II) can best be prepared by reaction of sulfamoyl-substituted benzyl lower alkyl ketone (III)

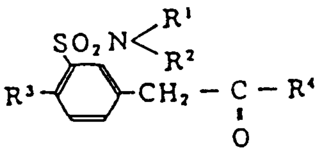

(III)

( wherein $R^1$, $R^2$, $R^3$ and $R^4$ are as defined above ), with optically active, substituted alkylamine (IV)

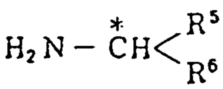

(IV)

( wherein $R^5$ and $R^6$ are as defined above ), in the presence of reducing agent. In the definition of groups herein, the term "lower" means, unless otherwise specified, linear or branched chains of 1 to 5 carbon atoms, preferably those containing no asymmetric carbon.

Thus, illustrative examples of lower alkyl groups include methyl, ethyl, propyl, isopropyl, butyl, isobutyl, tert-butyl, pentyl,isopentyl and neopentyl groups; and those of lower alkoxyl groups include methoxy, ethoxy, propoxy, isopropoxy, butoxy, isobutoxy, tert-butoxy, pentyloxy and isopentyloxy groups. Particularly, the lower alkyl represented by $R^5$ is preferably methyl, ethyl, isopropyl or isobutyl, most preferably methyl.

Typical examples of carboxy-lower-alkyl groups include carboxymethyl, carboxyethyl, carboxypropyl, carboxybutyl and carboxypentyl groups. As examples of lower-alkoxycarbonyl-lower-alkyl groups, may be mentioned esters of the above carboxy-lower-alkyls with a lower alkanol, such as methoxycarbonylmethyl, ethoxycarbonylmethyl, propoxycarbonylmethyl, isopropoxycarbonylmethyl, butoxycarbonylmethyl, isobutoxycarbonylmethyl, tert-butoxycarbonylmethyl, pentyloxycarbonylmethyl, methoxycarbonylethyl, ethoxycarbonylethyl, propoxycarbonylethyl, isopropoxycarbonylethyl, butoxycarbonylethyl, isobutoxycarbonylethyl, tert-butoxycarbonylethyl, pentyloxycarbonylethyl, methoxycarbonylpropyl, ethoxycarbonylpropyl, tert-butoxycarbonylpropyl, methoxycarbonylbutyl, ethoxycarbonylbutyl, tert-butoxycarbonylbutyl, methoxycarbonylpentyl, ethoxycarbonylpentyl and tert-butoxycarbonylpentyl.

"Decomposition" herein means a decomposition reaction in which $R^5$-$CH_2$-$R^6$ or $R^5$-CO-$R^6$ is stereospecifically eliminated from a compound (II), which includes "hydrogenesis" by the action of a reducing agent

Therefore, when $R^6$ is a substituted phenyl, the substituent should be such a group that does not adversely affect the hydrogenesis with the elimination of $R^5$-$CH_2$-$R^6$ but accelerates this elimination reaction. Illustrative examples of preferred substituents include nitro and halogens such as bromine.

"Hydrogenesis" is an advantageous method because it involves few reaction steps and high reaction and optical yields.

Shown below is the flow chart for the manufacturing process of this invention.

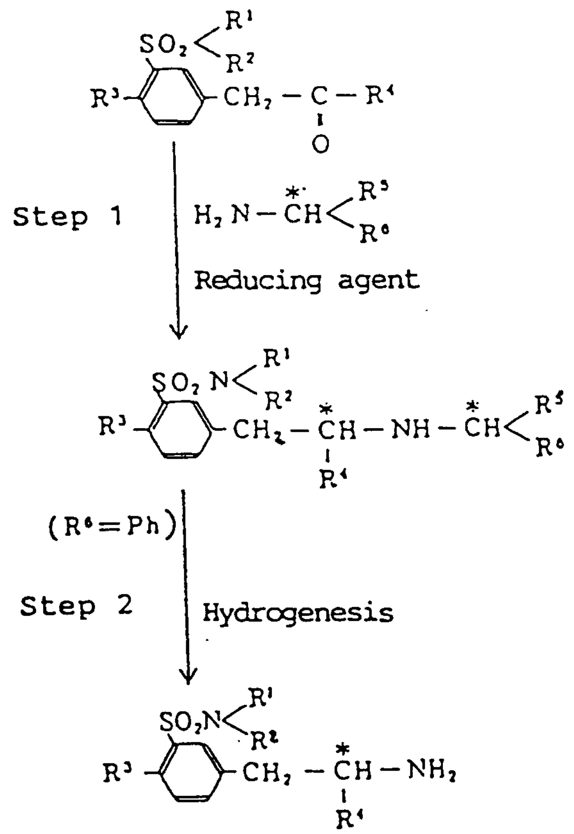


(III)

(IV)

(II)

(I)

( wherein $R^1$, $R^2$, $R^3$, $R^4$, $R^5$ and $R^6$ are as defined above and Ph denotes a substituted or unsubstituted phenyl).

Preferred details for each reaction step in the process of the invention are given below.

Step 1

Compound (II) is prepared by condensation between compound (III) and compound (IV) in the presence of reducing agent.

The reaction can be carried out in an organic solvent, such as methanol, ethanol or benzene, using the two reactants in nearly equimolar amounts ( or with a slight excess of either) one of them ), in the presence of lithium aluminum hydride, sodium cyanoborohydride, sodium borohydride or borane, or by catalytic reduction, under elevated pressure as required, in the presence of a catalyst such as Raney nickel, platinum/carbon, palladium/carbon and platinum oxide, or by electrolytic reduction using copper or platinum as cathode. Of the three modes of reduction mentioned above, catalytic reduction is the most preferred because of the ease of operation and higher optical yield.

The reaction temperature and time may vary depending on the type and quantity of reactants used, the type of reducing agent, pressure conditions and other factors. However, the reaction is generally carried out at room temperature or at elevated temperature, preferably at about 40 to 80 °C, for 1 to 48 hours under normal pressure and for a shorter time under an elevated pressure.

The pressure, if applied, is generally 1 to 100 atm, preferably 2 to 20 atm.

The above-described synthesis of secondary amines by reductive condensation between a carbonyl compound and an amine may be allowed to proceed in two separate steps: dehydration reaction ( for example, by the use of Dean-Stark trap ) to form a Schiff base, and reduction of the isolated Schiff base.

The reaction of this step preferentially gives a compound (II) in which the absolute conformation around the asymmetric carbon bonded to $R^4$ is the same as that around the asymmetric carbon bonded to $R^5$ and $R^6$ in the compound (IV) used as reactant. To be more specific, the reaction preferentially gives R,R-isomer or S,S-isomer.

The final compound (II) of this step, after being isolated or without being isolated, is submitted to the next step in the form of a free base or an acid addition salt.

Step 2

A compound (II) obtained in Step 1 can be converted to the corresponding compound (I) or a salt thereof by hydrogenesis.

Hydrogenesis is generally effected in a solvent, such as methanol or ethanol, by catalytic reduction using a theoretical amount of hydrogen gas in the presence of palladium/carbon as catalyst under elevated pressure as required. Catalytic reduction is most advantageous in this case because the final product can be obtained with a nearly quantitative yield. This reaction is carried out at room temperature or at elevated temperature, preferably at 40 to 80 °C. The reaction time, which depends on various reaction conditions, is generally 1 to 48 hours under normal pressure and is shorter under elevated pressure. The pressure, when applied, is usually from 1 to 100 atm.

The absolute conformation around the asymmetric carbon in the compound (I) thus obtained is identical to that around the asymmetric carbon bonded to $R^4$ in the compound (II), whether the two asymmetric carbon atoms in the compound (II) have same or different absolute conformation.

The compounds prepared by the method described above can be isolated and purified by commonly used chemical operations, such as filtration, crystallization and recrystallization.

The optically active benzenesulfonamide derivatives (I) are useful as antihypotensive agents and as intermediates for the manufacture of adrenergic receptor α-blocking agents having a side chain,

$$-CH_2\overset{*}{C}H-NH-,$$
$$\llcorner(\text{lower alkyl})$$

at the meta position which are described in Japanese Patent Publications No.110665 (1981) and No.136561 (1982).

The process of this invention is capable of producing optically active benzenesulfonamide derivatives (I) of high optical purity with higher reaction yield and optical yield, compared with the conventional method.

According to the conventional method, synthesis of one optical isomer of a compound (I) from compound (A-II) requires six reaction steps ( for hydrochloride ) or seven reaction steps ( for free base ), the overall reaction yield being about 10.5% for the former and about 9% for the latter.

In contrast, the process of this invention requires only two to four reaction steps to obtain one optical isomer of a compound (I) from a compound (III) ( two steps for hydrochloride when $R^6$ = Ph; three steps for hydrochloride when $R^6$ = Ph; and four steps for free base when $R^6$ = Ph ). The overall yield, although somewhat different depending on the synthetic route and reaction conditions, is about 51.3% for hydrochloride and about 43.0% for free base under optimum conditions a 4 to 5 times higher than by the conventional method.

In addition, the process of this invention gives final products of high optical purity, and hence the optical yield is also very high [up to 99.8%] - see Examples 1 to 3.

The process of this invention is simple in operation because of the few reaction steps involved, and still higher yields can be achieved by optimum selection of reaction conditions. The reactions involved proceed very smoothly even under mild conditions and use of reactants in larger quantities does not lead to drop in yield, making the process of this invention suitable for commercial production.

Compounds having adrenergic receptor α-blocking effect can be derived from a compound (I) according to the reaction given below,

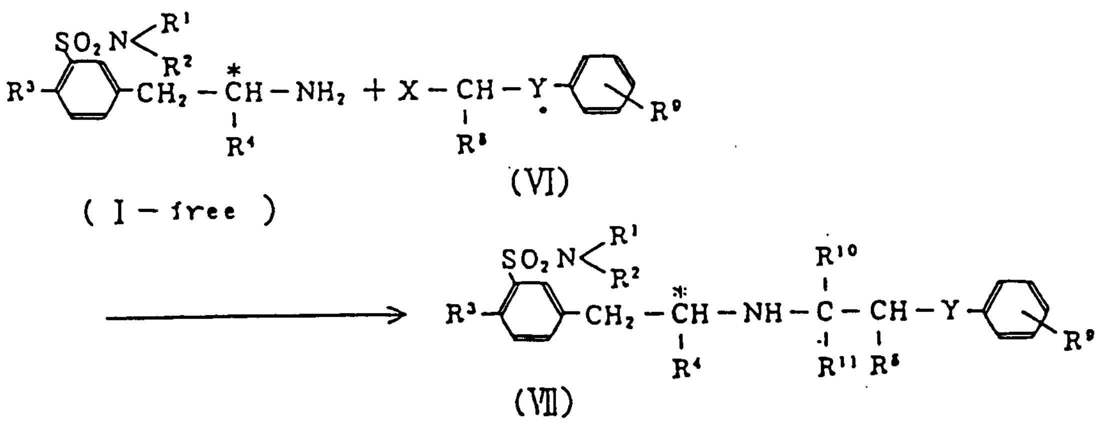

( I - free )

(VI)

(VII)

( wherein $R^1$, $R^2$, $R^3$ and $R^4$ are as defined above; X is a radical

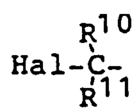

or a formyl group; Hal denotes a halogen atom; $R^8$, $R^{10}$ and $R^{11}$ are selected independently from a hydrogen atom and lower alkyl groups; Y is a methylene group or oxygen atom; and $R^9$ is a hydrogen atom or a lower alkyl, lower alkoxyl or lower alkenyloxy group ).

The halogen atom Hal may be iodine, bromine or chlorine, and the lower alkenyloxy group $R^9$ is, for example, vinyloxy, allyloxy, butenyloxy, isobutenyloxy or pentenyloxy.

The optically active m-(substituted-aminoalkyl)benzene-sulfonamides (VII) having adrenergic $\alpha$-blocking effect and useful as antihypertensive agents can be prepared by reaction of compound (I) ( in the form of free base ) with halide (IV), or by reductive condensation between compound (1) ( free base ) and formyl derivative of compound (VI).

It is preferable that the carbon atom to which $R^8$ is bonded and that to which $R^{10}$ and $R^{11}$ are bonded are not asymmetric carbon atoms. If either one of these is asymmetric an optically active compound (VI) previously isolated should be used for the reaction.

When X in compound (VI) is

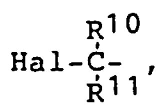

the reaction is preferably carried out in the absence of solvent or in an organic solvent ( e.g., benzene, toluene, xylene, dimethylformamide, dichloromethane, dichloroethane, methanol or ethanol ) at room temperature, at elevated temperature or under reflux, using an equal or slight molar excess amount of compound (VI).

In some instances, addition of secondary or tertiary amine ( e.g., pyridine, picoline, N,N-dimethylaniline, N-methylmorpholine, trimethylamine, triethylamine or dimethylamine ) or inorganic base ( e.g., potassium carbonate, sodium carbonate or sodium bicarbonate ) is effective in ensuring smooth reaction.

When a formyl derivative is used as compound (VI), the reaction may be carried out in much the same manner as in Step 1 of the process of this invention.

The process of this invention will become more apparent from the following Examples.

Example 1

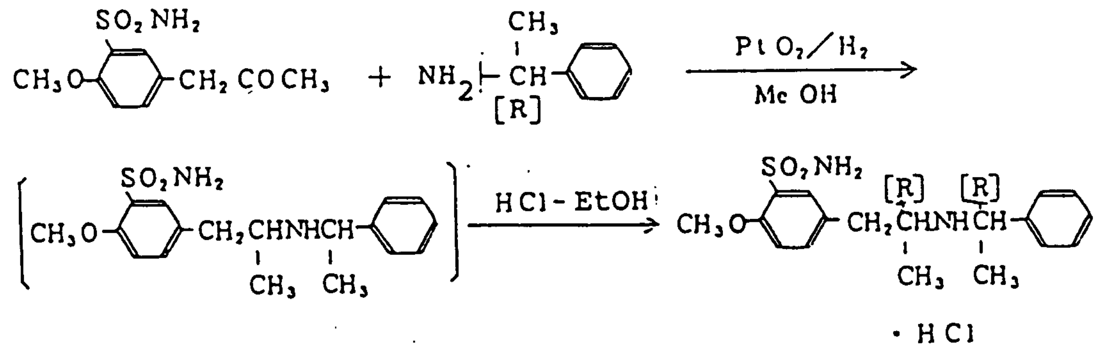


Method [A]

A mixture of 194.4 g 5-acetonyl-2-methoxybenzenesulfonamide, 96.8 g R-(+)-α-methylbenzylamine, 0.8 g platinum oxide and 4 liters of methanol was subjected to catalytic reduction at 50 to 52°C under normal pressure for 20 hours in a hydrogen atmosphere ( R/S ratio in the reaction solution: 85/15 ). At the end of reaction, the platinum oxide was filtered off, the filtrate was acidified by addition of an ethanolic solution of hydrogen chloride, and the solvent was distilled off under reduced pressure. Acetone ( 800 ml ) was added to the residue to effect crystallization, the resulting mixture was heated under reflux for one hour, and the crystals which separated out after cooling were collected, giving 250 g of crude 2R,1R-2-methoxy-5-[2-(1-methylbenzylamino)propyl]benzenesulfonamide hydrochloride. Optical purity of these crystals as R,R-isomer was 94.5%. Acetone ( 2.5 liters ) was added to the crude crystals thus obtained, the resulting suspension was heated under reflux for two hours, and the crystals were collected by filtration after cooling. This cycle of operations was repeated four times, affording 215 g of crystals with an optical purity of 98.0%.

The crystals were mixed with 80 ml water and 160 ml acetone, the mixture was brought into solution by heating, 1,850 ml acetone was added to the solution to separate out crystals, the resulting mixture was again heated under reflux for 30 minutes, and the crystals were collected by filtration after cooling. This cycle of operations was repeated three times, affording 164 g of pure crystals.

Yield; 53% Melting point: 232-236°C ( dec. )

$[\alpha]_D^{23}$ : +33.5 ( c = 1.04, MeOH )

| Elemental analysis ( $C_{18}H_{25}ClN_2O_3S$ ): | | | | | |
|---|---|---|---|---|---|
| | C(%) | H(%) | N(%) | Cl(%) | S(%) |
| Calcd. | 56.17 | 6.55 | 7.28 | 9.21 | 8.33 |
| Found | 56.10 | 6.59 | 7.30 | 9.29 | 8.31 |

Method [B]

A mixture of 24.3 g 5-acetonyl-2-methoxybenzenesulfonamide, 12.1 g R-(+)-α-methylbenzylamine, 15 g Raney nickel ( wet ) and 500 ml methanol was subjected to catalytic reduction at 50 to 52°C under normal pressure for 20 hours in a hydrogen atmosphere ( R/S ratio in the reaction solution: 92/8 ). At the end of reaction, the Raney nickel was filtered off, the filtrate was acidified by addition of an ethanolic solution of hydrogen chloride, and the solvent was distilled off under reduced pressure. Acetonitrile ( 200 ml ) was added to the residue to effect crystallization, the resulting mixture was heated under reflux for one hour, and the crystals which separated out after cooling were collected, giving 26.9 g of crude 2R,1R-2-methoxy-5-[2-(1-methylbenzylamino)propyl]benzenesulfonamide hydrochloride. Optical purity of these crystals as R,R-isomer was 98.3%. Water ( 11 ml ) and acetonitrile ( 22 ml ) were added to the crude crystals thus obtained, the resulting mixture was brought into solution by heating, acetonitrile ( 520 ml ) was added to effect crystallization, the mixture was heated under reflux for 30 minutes, and the crystals were collected by

filtration after cooling. This cycle of operations was repeated three times, affording 19.6 g of pure crystals. Yield: 51%

Method [C]

A mixture of 9.72 g 5-acetonyl-2-methoxybenzenesulfonamide, 4.84 g R-(+)-α-methylbenzylamine, 5 g Raney nickel ( wet ) and 200 ml methanol was subjected to catalytic reduction at 58 to 60°C under a pressure of 10 $Kg/cm^2$ for 20 hours in a hydrogen atmosphere ( R/S ratio in the reaction solution: 92/8 ). At the end of reaction, the Raney nickel was filtered off, the filtrate was acidified by addition of an ethanolic solution of hydrogen chloride, and the solvent was distilled off under reduced pressure. Acetonitrile ( 80 ml ) was added to the residue to effect crystallization, the resulting mixture was heated under reflux for one hour, and the crystals which separated out after cooling were collected, giving 11.8 g of crude 2R,1R-2-methoxy-5-[2-(1-methylbenzylamino)propyl]benzenesulfonamide hydrochloride. Optical purity of these crystals as R,R-isomer was 98.2%. Water ( 5 ml ) and acetonitrile ( 10 ml ) were added to the crude crystals thus obtained, the resulting mixture was brought into solution by heating, acetonitrile ( 220 ml ) was added to effect crystallization, the mixture was heated under reflux for 30 minutes, and the crystals were collected by filtration after cooling. This cycle of operations was repeated three times, affording 8.31 g of pure crystals. Yield: 54%

The crystals of 2R,1R-2-methoxy-5-[2-(1-methylbenzylamino)propyl]benzenesulfonamide hydrochloride obtained above by methods [A], [B] and [C] were each subjected to high-performance liquid chromatography [ column: YM C-Pack A- 302 ( ODS ), 15cm x 4.6mm ID; column temperature: room temperature; mobile phase: aqueous solution of $K_2HPO_4$(1.0g) and $KH_2PO_4$(1.0g)/methanol/tetrahydrofuran ( 65:30:5 v/v ); flow speed: 1 ml/min; UV detector: λ, 285 nm )]. The optical purity of 2R,1R-2-methoxy-5-[2-(1-methylbenzylamino)propyl]-benzenesulfonamide hydrochloride showing a retention time of the seven minutes was 99.8% or higher.

Example 2

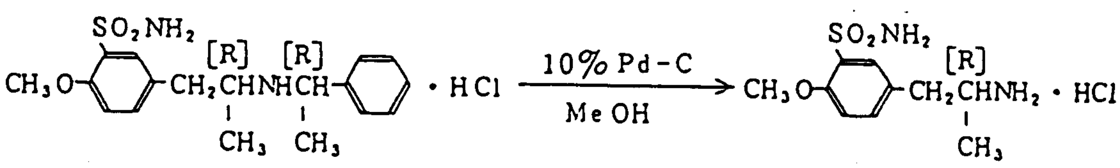


A mixture of 560 ml methanolic solution containing 28 g 2R,1R-2-methoxy-5-[2-(1-methylbenzylamino)-propyl]benzenesulfonamide hydrochloride ( optical purity: 99.8% or higher ) and 2.8 g of 10% palladium/carbon was subjected to catalytic reduction at 45 to 50°C under normal pressure in a hydrogen atmosphere until the theoretical volume of hydrogen was consumed. The palladium/carbon catalyst was filtered off, the solvent was distilled off under reduced pressure, and the crystals which separated out were washed with acetone, affording 19.4 g of (R)-(-)-5-(2-aminopropyl)-2-methoxybenzenesulfonamide hydrochloride.

Yield: 95% Melting point: 276-278°C ( dec. )

$[\alpha]_D^{22}$ :-7.1 ( c = 1.0, MeOH )

| Elemental analysis ( $C_{10}H_{17}ClN_2O_3S$ ): | | | | | |
|---|---|---|---|---|---|
| | C(%) | H(%) | N(%) | Cl(%) | S(%) |
| Calcd. | 42.78 | 6.10 | 9.98 | 12.63 | 11.42 |
| Found | 42.82 | 5.96 | 9.71 | 12.56 | 11.40 |

One milligram of (R)-(-)-5-(2-aminopropyl]-2-methoxybenzenesulfonamide hydrochloride obtained above was taken in a microtube, and 100 µl of 5% triethylamine solution in N,N-dimethylformamide was added to dissovle the hydrochloride. To this solution was added 300 µl of MCF reagent ( 0.1M solution of (-)-menthyl chloroformate in toluene; Legis Corp. ), and the reaction was allowed to proceed for 30 minutes with

occasional stirring.

The reaction was terminated by addition of 50 µl methanol, and the reaction mixture was subjected to high-performance liquid chromatography [ column: Nucleosyl® 5 $C_{18}$, 305cm x 4.6mm ID; column temperature: room temperature; mobile phase: acetonitrile/methanol/0.01M $KH_2PO_4$ solution ( 50:150:40 v/v ); flow speed: 1 ml/min; UV detector: λ, 284 nm )]. The optical purity of (R)-(-)-5-(2-aminopropyl)-2-methoxybenzenesulfonamide hydrochloride showing a retention time of 20 minutes was 99.8% or higher.

Example 3

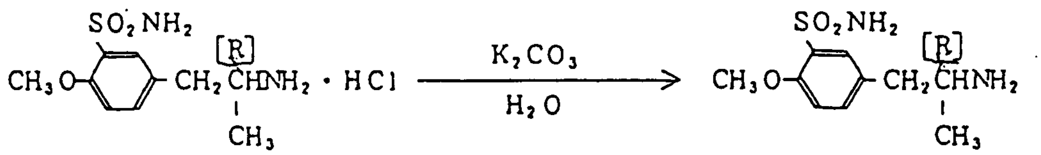


Method [A]

(R)-(-)-5-(2-aminopropyl)-2-methoxybenzenesulfonamide hydrochloride ( 20 g ) was dissolved in 140 ml water, and 50 ml of saturated potassium carbonate solution was added. After crystals separated out, the mixture was stirred at room temperature for two hours, and the crystals were collected by filtration and recrystallized from 75 ml water, affording 14.2 g of (R)-(-)-5-(2-aminopropyl)-2-methoxybenzenesulfonamide.

Yield: 82% Melting point: 166-167 °C ( dec. )

$[\alpha]_D^{23}$ : -17.3 ( c = 1.07, MeOH )

| Elemental analysis ( $C_{10}H_{16}N_2O_3S$ ): | | | | |
|---|---|---|---|---|
| | C(%) | H(%) | N(%) | S(%) |
| Calcd. | 49.16 | 6.60 | 11.47 | 13.12 |
| Found | 49.08 | 6.49 | 11.26 | 13.02 |

Method [B]

(R)-(-)-5-(2-aminopropyl)-2-methoxybenzenesulfonamide hydrochloride ( 4.31 g ) was dissolved in 30 ml water, 21 g anhydrous potassium carbonate was added to the solution, the mixture was stirred at room temperature for two hours, and the crystals which separated out were collected by filtration and recrystallized from 20 ml water, affording 3.14 g of (R)-(-)-5-(2-aminopropyl)-2-methoxybenzenesulfonamide.

Yield: 84%.

Example 4

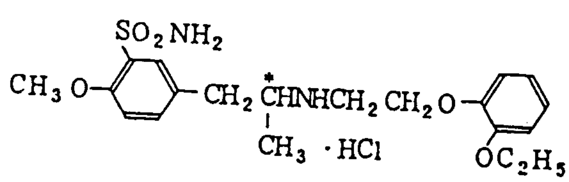

2.4 g of R(-)-5-[(2-amino-2-methyl)ethyl]-2-methoxybenzenesulfonamide (namely, (R)-(-)-5-(2-aminopropyl)-2-methoxybenzenesulfonamide) and 1.2 g of 2-(o-ethoxyphenoxy)-ethylbromide were dissolved in 120 ml of ethanol, and the mixture was refluxed for 16 hours. The solvent was distilled away, and the residue was alkalified with 10 % aqueous sodium hydroxide. The separated oily material was extracted with ethyl acetate, and the extract solution was washed with saturated aqueous sodium chloride, and dried over

anhydrous magnesium sulfate. The solution was subjected to distillation, and the residue was subjected to silica gel column chromatography, eluted with chloroform-methanol (9:5) to give 1.5 g of crude crystals.

The crystals were treated with ethanolic hydrochloric acid to give R(-)-5-[2-[[2-(o-ethoxyphenoxy)ethyl]-amino]-2-methylethyl]-2-methoxybensenesulfonamide hydrochloride.
Melting point: 228-230 ° C

| Elemental analysis ($C_{20}H_{29}ClN_2O_5S$): | | | |
|---|---|---|---|
| | C(%) | H(%) | N(%) |
| Calcd.<br>Found | 53.99<br>53.90 | 6.57<br>6.64 | 6.30<br>6.27 |

$[\alpha]_D^{24}$ : -4.0 ° (c = 0.35, methanol)

## Claims

**1.** A process for producing optically active benzenesulfonamide derivative (I)

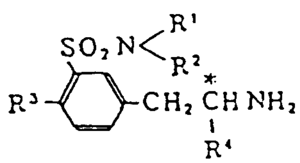

(I)

which comprises decomposing m-(2-substituted-alkylaminoalkyl)benzenesulfonamide derivative (II) by hydrogenesis

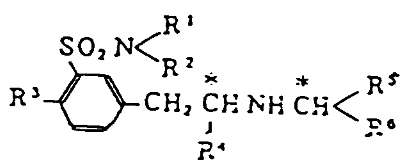

(II)

wherein $R^1$ and $R^2$ are selected independently from a hydrogen atom and $C_1$-$C_5$ alkyl groups; $R^3$ is a hydrogen atom or a $C_1$-$C_5$ alkyl, hydroxyl or $C_1$-$C_5$ alkoxyl group; $R^4$ is a $C_1$-$C_5$ alkyl group; $R^5$ is a $C_1$-$C_5$ alkyl, carboxy-($C_1$-$C_5$ alkyl) or ($C_1$-$C_5$ alkoxy)carbonyl-($C_1$-$C_5$ alkyl)group; and $R^6$ is a substituted or unsubstituted phenyl group.

**2.** A process according to claim 1 wherein the compound (II) is obtained by a method which comprises reacting sulfamoyl-substituted benzyl lower alkyl ketone (III)

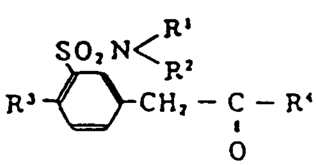

(III)

with optically active, substituted alkylamine (IV)

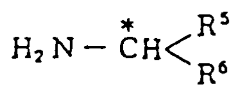

(IV)

in the presence of reducing agent.

**3.** A process according to claim 1 or 2 wherein product compound (I) is further reacted as follows :

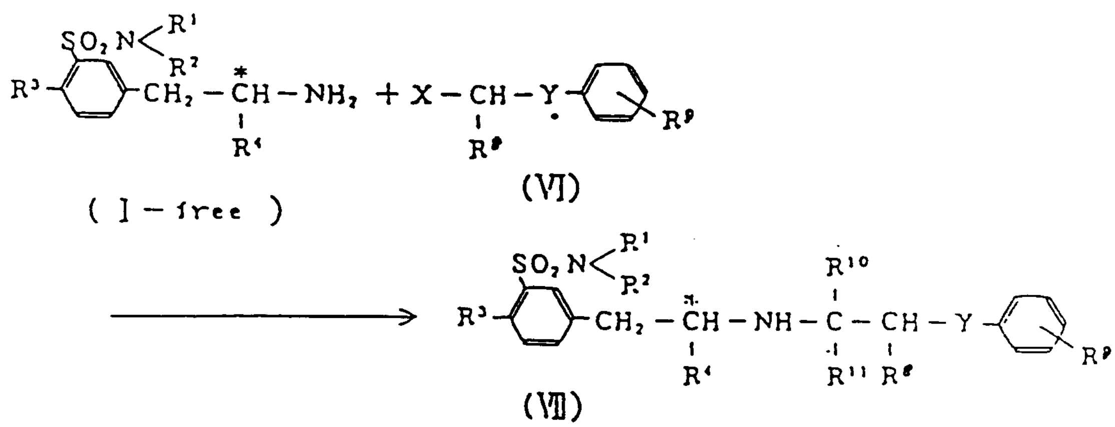

( I – free )

(VI)

(VII)

wherein X is

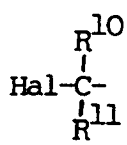

or a formyl group; Hal is a halogen atom; $R^8$, $R^{10}$ and $R^{11}$ are selected independently from a hydrogen atom and $C_1$-$C_5$ alkyl groups; Y is a methylene group or oxygen atom; and $R^9$ is a hydrogen atom or a $C_1$-$C_5$ alkyl, $C_1$-$C_5$ alkoxy or $C_1$-$C_5$ alkenyloxy group.

**4.** A process according to any preceding claim wherein the decomposition is by stereospecific elimination $R^5$-$CH_2$-$R^6$ or $R^5$-CO-$R^6$ from compound II.

**5.** A process according to claim 4 wherein the decomposition is effected by reduction of compound I when $R^6$ is phenyl or substituted phenyl.

## Patentansprüche

**1.** Verfahren zur Herstellung optisch aktiver Benzolsulfonamid Derivate (I)

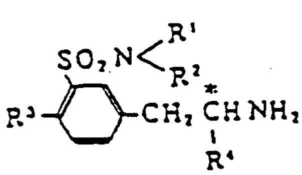

( I )

welches umfasst, zerlegen eines m-(2-substituierter-Alkylaminoalkyl)benzolsulfonamid Derivates (II) durch Hydrospaltung

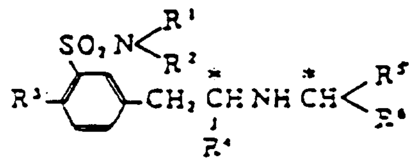

(II)

worin $R^1$ und $R^2$ unabhängig voneinander ausgewählt sind von einem Wasserstoffatom und $C_1$-$C_5$ Alkylgruppen; $R^3$ ist ein Wasserstoffatom oder eine $C_1$-$C_5$ Alkylgruppe, Hydroxyl oder $C_1$-$C_5$ Alkoxygruppe; $R^4$ ist eine $C_1$-$C_5$ Alkylgruppe; $R^5$ ist eine $C_1$-$C_5$ Alkyl, Carboxy-($C_1$-$C_5$ Alkyl) oder ($C_1$-$C_5$ Alkoxy)carbonyl-($C_1$-$C_5$ Alkyl)gruppe; und $R^6$ ist eine substituierte oder unsubstituierte Phenylgruppe.

2. Verfahren gemäss Anspruch 1, dadurch gekennzeichnet, dass die Verbindung (II) durch ein Verfahren erhalten wird, welches die Reaktion eines Sulfamoyl substituierten Benzylniederalkylketons (III)

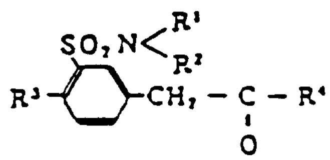

(III)

mit einem optisch aktiven substituierten Alkylamin (IV)

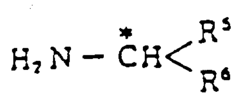

(IV)

in Gegenwart eines Reduktionsmittels umfasst.

3. Verfahren gemäss Anspruch 1 oder 2, worin die Produkteverbindung (I) wie folgt weiter zur Reaktion gebracht wird:

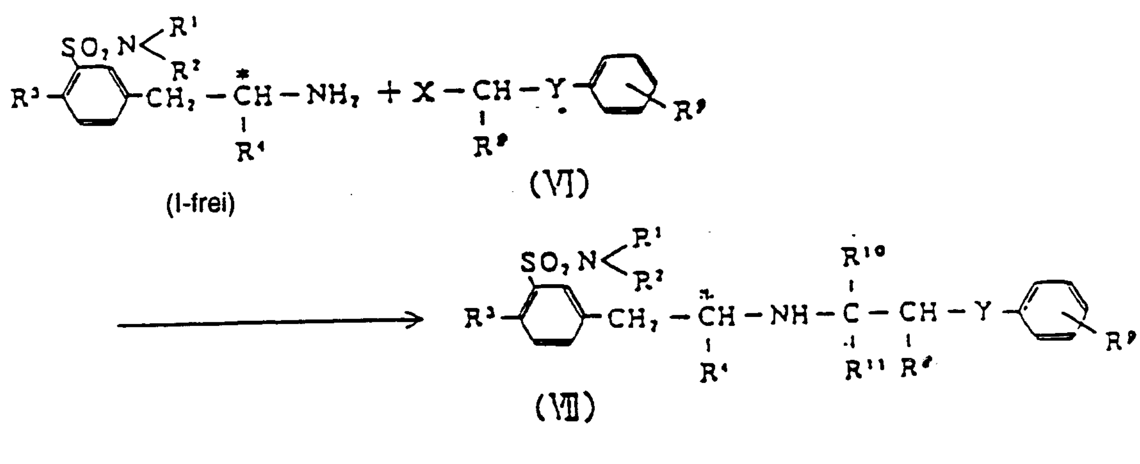

(I-frei)

(VI)

(VII)

worin X

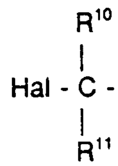

oder eine Formylgruppe ist; Hal bedeutet ein Halogenatom; $R^8$, $R^{10}$ und $R^{11}$ sind unabhängig voneinander ausgewählt von einem Wasserstoffatom und $C_1$-$C_5$ Alkylgruppen; Y ist eine Methylengruppe oder ein Sauerstoffatom; und $R^9$ ist ein Wasserstoffatom oder eine $C_1$-$C_5$ Alkyl, $C_1$-$C_5$ Alkoxy oder $C_1$-$C_5$ Alkenyloxygruppe.

**4.** Verfahren gemäss jedem vorausgehenden Anspruch, dadurch gekennzeichnet, dass die Zerlegung durch eine stereospezifische Abspaltung von $R^5$-$CH_2$-$R^6$ oder $R^5$-CO-$R^6$ aus der Verbindung II erfolgt.

**5.** Verfahren gemäss Anspruch 4, dadurch gekennzeichnet, dass die Zerlegung durch Reduktion der Verbindung I bewirkt wird, wenn $R^6$ Phenyl oder substituiertes Phenyl bedeutet.

## Revendications

**1.** Un procédé de production d'un dérivé de benzènesulfonamide optiquement actif (I)

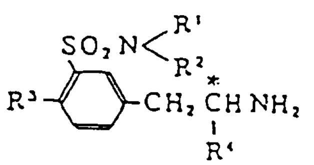

(I)

qui comprend la décomposition d'un dérivé de m-(alkyl substitué en 2-aminoalkyl)benzène sulfonamide (II) par hydrogénation

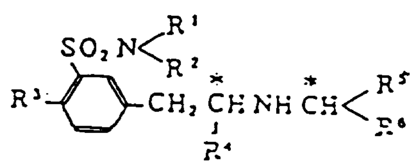

(II)

dans lequel $R^1$ et $R^2$ sont choisis indépendamment parmi un atome d'hydrogène et des groupes alkyle en $C_1$-$C_5$ ; $R^3$ est un atome d'hydrogène ou un groupe alkyle en $C_1$-$C_5$, hydroxyle groupe alcoxyle en $C_1$-$C_5$ ; $R^4$ est un groupe alkyle en $C_1$-$C_5$ ; $R^5$ est un groupe alkyle en $C_1$-$C_5$, carboxy (alkyle en $C_1$-$C_5$) ou (alcoxy en $C_1$-$C_5$) carbonyl-(alkyle en $C_1$-$C_5$) ; et $R^6$ est un groupe phényle substitué ou non substitué.

**2.** Un procédé conforme à la revendication 1,
dans lequel le composé (II) est obtenu par une méthode qui comprend la réaction d'une benzylalkyl-inférieur cétone substituée par un sulfamoyle (III)

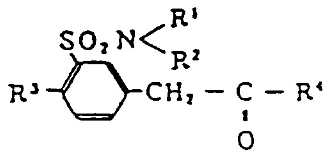

(III)

avec une alkylamine substituée optiquement active (IV)

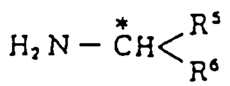

(IV)

en présence d'un agent réducteur.

**3.** Un procédé conforme à la revendication 1 ou 2, dans lequel le composé de produit (I) est en outre soumis à une réaction comme suit:

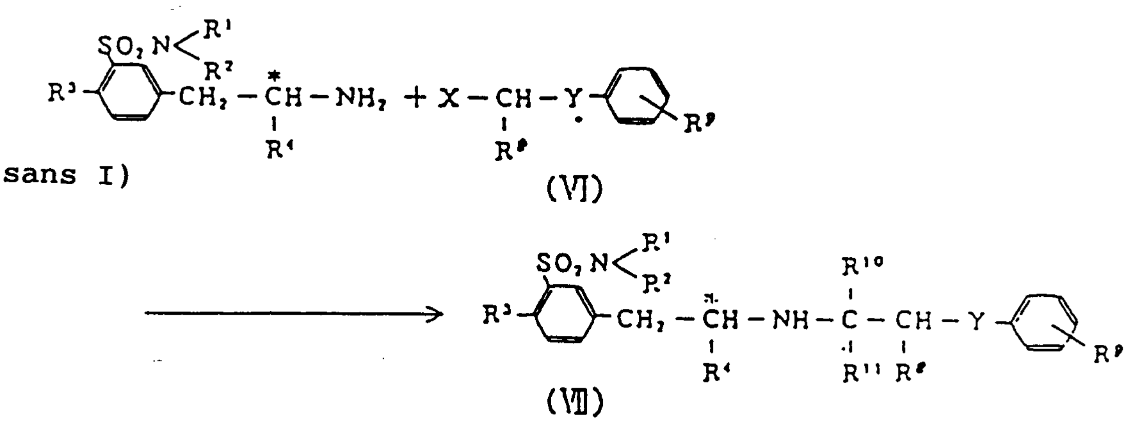

(sans I)

(VI)

(VII)

dans lequel X est un groupe

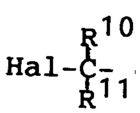

ou un groupe formyle ; Hal est un atome d'halogène ; $R^8$, $R^{10}$ et $R^{11}$ sont choisis indépendamment parmi un atome d'hydrogène et des groupes alkyle en $C_1$-$C_5$ ; Y est un groupe méthylène ou un atome d'oxygène ; et $R^9$ est un atome d'hydrogène ou un groupe alkyle en $C_1$-$C_5$, alcoxyle en $C_1$-$C_5$ ou alcényloxy en $C_1$-$C_5$.

**4.** Un procédé conforme à toute revendication précédente, dans lequel la décomposition s'effectue par élimination stéréospécifique de $R^5$-$CH_2$-$R^6$ ou $R^5$-CO-$R^6$ à partir du composé II.

**5.** Un procédé conforme à la revendication 4, dans lequel la décomposition est effectuée par réduction du composé I quand $R^6$ est un phényle ou un phényle substitué.